# EUROPEAN PATENT APPLICATION

(11) **EP 4 803 046 A1**
(43) Date of publication of application: **09.09.2026**
(21) Application number: 26160777.4
(22) Date of filing: 25.02.2026
(51) Int. Cl.: A61F 2/30

(54) **DISSOLVABLE PORE REINFORCEMENT FOR SURFACE OVERMOLDING**

(30) Priority: 03.03.2025 US 202563766097 P
(71) Applicant: Howmedica Osteonics Corp., Mahwah, NJ 07430 (US)
(72) Inventor: WELLER, Daniel, Cedar Grove, New Jersey, 07009 (US); HANSON, Keenan Michael, Sloatsburg, New York, 10974 (US)
(74) Representative: ABG Intellectual Property Law, S.L.

(57) **Abstract**

A method of manufacturing an implant (200) including additively manufacturing the implant (200) from a first material such that the implant (200) defines a plurality of pores (205). Then inserting a filler material (206) into the pores (205) after the implant (200) has been manufactured. Next, overmolding the implant (200) with a second material after the filler material (206) has been inserted the pores (205), and then dissolving the filler material (206) from the pores (205) prior to deployment of the implant (200).

## Description

### BACKGROUND

In the field of orthopedic implants, various advancements have been made to improve the results brought about by the use of such implants. For example, complex porous structures, typically made from biocompatible metal such as titanium, are often utilized as bone ingrowth surfaces having an open scaffold-like space for cell growth and regeneration. While these complex structures are difficult to make with traditional manufacturing techniques, thanks to advancements in additive manufacturing, implants with these porous structures are more readily available and widely used in the medical procedures.

Another advancement is the use of poly-ether ether ketone ("PEEK") in implants to promote better bone health by reducing stress shield. Combining the benefits of the porous ingrowth structures and PEEK would enhance the effectiveness of various types of implants. However, polymeric components, such as those made of PEEK, are formed by injection molding at high pressures which can damage or deform the porous ingrowth structures.

Accordingly, there remains a need for manufacturing techniques that enable the integration of porous ingrowth structures with polymeric components.

### BRIEF SUMMARY

In one aspect, the present disclosure relates to manufacturing solutions that overcome the problems discussed above to provide, for example, an efficient process for manufacturing a composite implant having a PEEK body and a porous base, e.g., a titanium core, that would simultaneously provide sufficient strength and adequate bone fixation.

To combine these features, porous ingrowth surface structures may be 3D printed and then filled with a dissolvable support filler to create a solid preform with all voids or openings of the porous ingrowth filled by the filler. These solid preforms may be then overmolded with another material (e.g., PEEK) using injection molding techniques. Once the overmolding is completed, the support filler may be dissolved by exposing the pores of the porous bone ingrowth surface to a liquid substance.

In another aspect, the present disclosure relates to an implant manufactured by a process. In this process, the implant may be additively manufactured from a first material, and the implant may define at least one pore. Then a filler material may be inserted into the at least one pore after the implant has been additively manufactured. The implant may be overmolded with a second material after the filler material has been inserted into the at least one pore. The filler material may be removed (e.g., dissolved) from the at least one pore prior to implanting the implant. The implant may be submerged in water to remove the filler material. The process may further include a step of removing any second material covering the filler material after the dissolving step. The filler material may be packed into the at least one pore. The at least one pore may be completely filled with the filler material. The filler material may include a soluble salt. The filler material may include a ceramic material. The ceramic material may include a calcium sulfate and/or magnesium sulfate. The first material may be a biocompatible metal. The second material may be poly-ether ether ketone (PEEK) or another polymer. The overmolding step may use injection molding techniques to apply the second material.

In accordance with another aspect, the present disclosure relates to an implant manufactured by a process. In this process, a porous structure of the implant may be manufactured from a first material, and the porous structure may define openings extending therethrough. At least one opening of the porous structure may be filled with a filler material. The porous structure may be overmolded with a second material after the at least one opening is filled with the filler material. The filler material may be dissolved or removed via liquid suspension before the implant is implanted. In such instances, the implant may be submerged in a liquid solution. The second material may be overmolded on one side of the porous structure. The porous structure may be a bone ingrowth surface especially adapted to facilitate regrowth of bone tissue. The filler material may be adapted to withstand temperatures greater than 350° C and dissolvable in a liquid.

In accordance with yet another aspect, the present disclosure relates to an implant manufactured by a process. In this process, a porous structure defining a plurality of pores may be provided. The pores of the porous structure may be filled with a filler material. The porous structure may be made from a first material. A side of the porous structure may be overmolded with a second material. The filler material may be removed before deployment of the device. The filler material may be dissolved and/or dispersed with a liquid substance. The porous structure may be three-dimensional printed as part of the process. The second material may be overmolded at a pressure ranging between 300 bar to 2,000 bar.

In accordance with yet another aspect, the present disclosure relates to an implant manufactured by a process. In this process, the implant may be additively manufactured from a metallic material, and the implant may have a bone ingrowth surface defining a plurality of pores. The pores of the implant may be filled with a filler material after the implant has been additively manufactured. A first side of the implant may be overmolded with a material (e.g., a polymeric material) after the plurality of pores have been filled with the filler material. In such instances, the second side may not be overmolded such that it remains clear of any overmolded material. The second side may be opposite from the first side. The filler material may be dissolved and/or dispersed from the plurality of pores prior to implanting the implant and after the overmolding step has been completed. The filler material may be configured to dissolve and/or disperse in a liquid. The implant may be submerged into and/or washed with the liquid.

### BRIEF DESCRIPTION OF THE DRAWINGS

A more complete appreciation of the subject matter of the present disclosure and the various advantages thereof may be realized by reference to the following detailed description which refers to the accompanying drawings, in which:
FIG. 1A is a perspective view of an implant in accordance with an aspect of the present disclosure;
FIG. 1B is a close-up view of a porous structure of the implant of FIG. 1A;
FIG. 1C illustrates the porous structure of FIG. 1B during a process for fabricating the implant of FIG 1A in accordance with an aspect of the present disclosure;
FIGS. 2A-2D illustrate sequential steps for overmolding a porous structure in accordance with an aspect of the present disclosure; and
FIG. 3 is a diagrammatic view of a process for fabricating an implant in accordance with an aspect of the present disclosure.

### DETAILED DESCRIPTION

The present disclosure relates to methods for manufacturing items having a porous structure and a solid molded structure. The methods and components described herein will be discussed in the context of making an enhanced orthopedic implant. However, it should be noted that these methods and their corresponding structures may be used in other ways including, but not limited to, manufacturing components for industrial, laboratory, construction applications and the like. Additionally, it should be understood that this disclosure may apply to a variety of different implants including, but not limited to, knee implants, shoulder implants, hip implants, wrist implants, spine implants, dental implants, etc.

As mentioned above, PEEK implants (or implants that include PEEK) have benefits over traditional implants (e.g., PEEK knee implants vs. traditional knee implants) including better bone health due to reduced stress shielding. And porous structures (e.g., 3D printed porous structures) provide enhanced bone ingrowth surfaces that allow for cementless implants that frequently result in improved patient outcome and satisfaction over cemented implants. The present disclosure describes ways to combine the benefits of porous ingrowth surfaces with the bone health benefits of PEEK overmolded implants. By integrating these features, the present disclosure allows for the creation of implants that balance structural integrity, biological compatibility, and mechanical performance. The porous structure can facilitate osseointegration, enabling direct bone-implant bonding without reliance on bone cement, while the PEEK overmold can enhance fatigue resistance and durability. Additionally, by leveraging advanced manufacturing techniques, such as additive manufacturing and precision overmolding disclosed herein, the methods disclosed here provides design flexibility, allowing for patient-specific geometries that can optimize joint function and load distribution. As a result, these implants can improve long-term fixation, reduce the risk of implant loosening, and enhance overall surgical outcomes, making them particularly advantageous for orthopedic applications where stability and durability are critical.

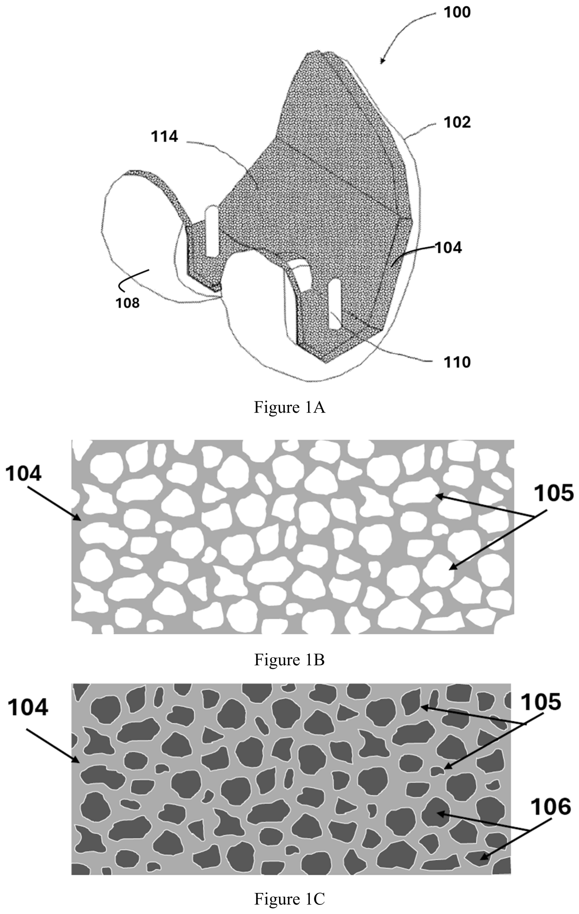

Now referring to FIGS. 1A-1C, a composite implant 100, according to an embodiment of the present disclosure, is a femoral component for a distal end of a femur that includes solid body 102 and porous structure 104 including pores 105. In some instances, such as in the example shown, articulating second surface 108 may contact or otherwise engage with another prosthesis, including but not limited to a tibial base plate. In other instances, second surface 108 may contact or otherwise engage with a bone, including but not limited to a tibia. Such contacts or engaging may take on any suitable arrangement that is appropriate for a particular application. In the illustrated embodiment, composite implant 100 has two securing posts 110 which may aid in the fixation of composite implant 100 to a recipient bone. Body 102 may be made of a material such as certain of those set forth below, e.g., PEEK, and may be molded over porous structure 104.

Porous structure 104 is made of a single construct, but it is contemplated that porous structure 104 may constitute a variety of separate porous structures (e.g., pores 105) to facilitate greater flexibility in combining a flexible outer body with a rigid porous structure, i.e., reducing or increasing the rigid porous structures based on the overall stiffness required for composite implant 100. Porous structure 104 has a bone-contacting surface 114 and an opposing surface (not shown) in contact with body 102.

Pores 105 of the porous structure 104 are designed to be filled with a temporary filler material during fabrication of the composite implant 100 which is later removed from the pores. For example, as shown in FIG. 1C, pores 105 of the porous structure 104 may be temporarily filled with a filler material 106 that is removable from the pores. When pores 105 are completely filled with filler material 106, the filler material provides internal structural reinforcement to porous structure 104 and prevents other material from entering pores 105 during various fabrication processes (e.g., overmolding body 102 around a portion of porous structure 104). In this manner, the porous structure may undergo various molding steps to form other segments of the composite implant without the pores being clogged with a molded material, or without the pores collapsing under external forces applied to the porous structure during a molding process. Methods for filling and removing filler material from pores of porous structures is discussed in more detail below. Additionally, these molded body structures and porous structures shown and discussed in the present disclosure may be made by the manufacturing processes discussed herein.

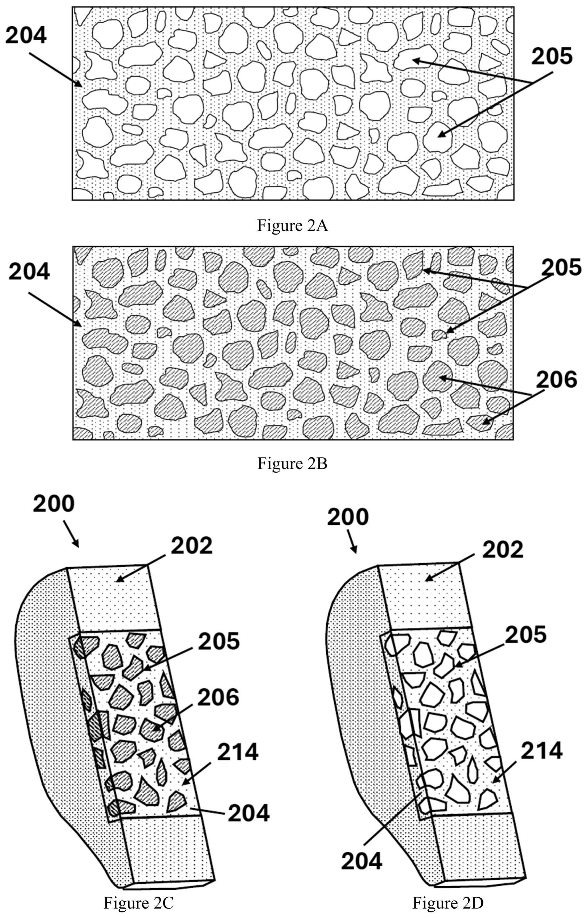

Referring now to FIGS. 2A-2D, there is shown sequential steps for making a composite implant 200 having a body 202 and a porous structure 204, according to an embodiment of the present disclosure. As shown in FIG. 2A, porous structure 204 includes pores 205 or openings defined by the porous structure that extend therethrough. In FIG. 2B, pores 205 are filled with filler material 206 such that the filler material fills or plugs most all the openings within porous structure 204. Once porous structure 204 is filled, the porous structure is partially overmolded by a molten material to form body 202. This is done such that a bone contacting side 214 of porous structure 204 is not covered by body 202 and continues to have external exposure as shown in FIGS. 2C and 2D.

In one example, the overmolding is achieved by placing porous structure 204 into a mold cavity of an injection molding machine or press. Then pressurized and molten PEEK, or another suitable polymer material as described herein, is injected into the mold configured to form body 202. Because filler material 206 fills or plugs most or all the openings in porous structure 204, it serves as a temporary structural reinforcement, ensuring that the polymeric material is precluded or prevented from entering pores 205 of the porous structure. Additionally, filler material 206 provides structural support to porous structure 204 during the formation of body 202 to prevent the porous structure from deforming or warping under the high heat and pressure of the injection molding process. Without this reinforcement, the porous structure could experience localized deformation, pore collapse, or distortion, leading to compromised mechanical performance and reduced bone ingrowth potential. The incompressibility and heat resistance of filler material 206 allow it to function as a rigid internal support, counteracting the force exerted by molten PEEK or other high-viscosity polymers during molding. This prevents pore constriction, collapse, or unintended polymer infiltration, which could otherwise negatively impact the overall porosity and biological performance of the implant.

Once body 202 has been formed, filler material 206 is removed from porous structure 204, as shown in FIG. 2D. This may be done, for example, by submerging and/or washing composite implant 200 into a water or a liquid solution bath. In some instances, composite implant 200 may be soaked a liquid reservoir for several minutes or several hours depending on the composition of filler material 206. In some instances, the liquid may be heated and/or agitated to enhance or expedite the removal of the pore filler.

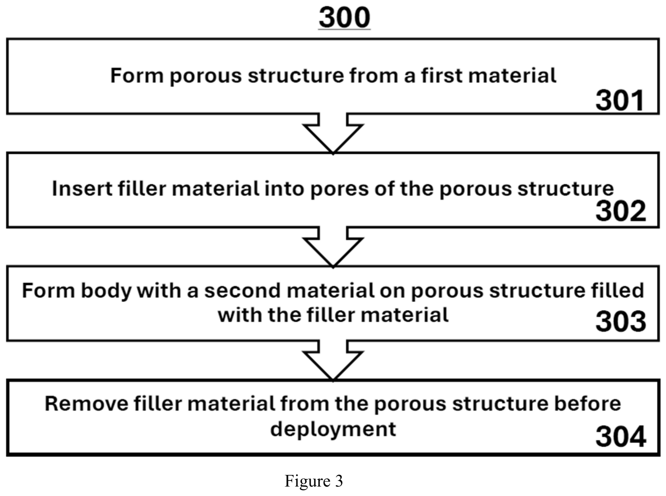

As depicted in FIG. 3, an implant may be fabricated with a fabrication process 300, according to an embodiment of the present disclosure. First, a porous structure is formed 301, which may be done through various manufacturing techniques, e.g., but not limited to, additively manufacturing techniques. In some instances, for example, the porous structure may be made from titanium, stainless steel, or other metallic materials and fabricated by utilizing any of the following additive manufacturing processes: (1) beam overlap fabrication using direct laser remelting in a cross section of a layer of metallic powder on a build platform with the power, speed, spot size of the laser coordinated so that a predetermined porosity of the metallic powder can be achieved, as disclosed in U.S. Patent Publication No. 2004/0191106; (2) tessellated unit cell fabrication that includes the steps of depositing a first layer of metal powder, scanning the first layer of metal powder with a laser beam to form a portion of a plurality of predetermined unit cells, and then depositing at least one additional layer of metal powder onto a previous layer by repeating the step of scanning a laser beam for the additional layers in order to continuing forming the predetermined unit cells, as disclosed in U.S. Patent Publication No. 2006/0147332; (3) laser and e-beam polymer interdigitation disclosed in U.S. Patent Publication No. 2007/0142914; or (4) conformal surfaces fabrication using a component file including a three-dimensional porous structure in a CAD volume having a boundary and a space populated with unit cells having a plurality of struts having nodes on each end, as disclosed in U.S. Patent Publication No. 2013/0268085, the disclosures of all of which are hereby incorporated by reference herein as if fully set forth herein. Additionally, the porous structures shown and discussed herein may be made in the manner disclosed by U.S. Pat. No. 8,350,186, the disclosure of which is hereby incorporated by reference herein as if fully set forth herein.

In other instances, the porous structure may be fabricated with other manufacturing techniques using sacrificial inserts that impart complex texture and porosity to injection molded articles as disclosed in U.S. Patent No. 9,370,609, the disclosure of which is hereby incorporated by reference herein. In such instances, the sacrificial inserts are removed from the molded porous structures by dissolution or decomposition without leaving behind residual material in the pores thereof. The porous structure may be, but is not limited hereto, made as a single construct that covers substantially the entire body (as shown for example in FIGS. 1A and 1B), or composed of multiple segments that partially cover the body. Other suitable biocompatible materials as described herein may also be used depending on the desired porosity of the porous structure.

Once the porous structure is formed, its pores are filled 302 with filler material which includes partially or entirely filling the pores. For example, the porous structure may be completely filed with filler material to prevent any material from entering therein during subsequent fabrication steps, e.g., injection molding. Alternatively, some pores may be partially filled with the filler material to allow the molten material fill portions of the porous structure and then solidify therein to form a strong connection between porous structure and the molded body structure. Filling the pore or pores of the porous structure may also include packing or compacting the filler material into the pore or pores to eliminate any voids or spaces that may exist between segments of the filler material.

The filler material can be a dissolvable material having high structural rigidity and capable of withstand high-pressure (e.g., between 300 bar and 2,000 bar) and high-heat (e.g., between 300° F and 800° F) conditions without melting or deteriorating. In some instances, for example, the filler material may include various salts such as those containing elements like sodium (Na), calcium (Ca), magnesium (Mg) and/or potassium (K), like sodium chloride, calcium sulfate, magnesium sulfate and potassium chloride. For example, the filler material may be a calcium sulfate plaster that is formulated to be water soluble or dissolvable in an acid. In other instances, the filler material may include ceramics such as silica that can be dissolved certain solutions or acid baths, as disclosed in U.S. Patent No. 4,569,384, the disclosure of which is hereby incorporated by reference herein.

Once filled with filler material, the porous structure undergoes a high-heat and high-pressure injection molding process during which a body or solid structure is molded 303 over one or more sides of the porous structure. But at least one or more sides of the porous structure will not be covered by the overmolded structure to facilitate removal of the filler material post-molding and to provide a bone contacting surface adapted for tissue ingrowth (as shown in FIGS. 1 and 2D).

The filler material is then removed 304 from the porous structure by selective dissolution, liquid suspension, decomposition, or a combination thereof without leaving behind residual material that could contaminate the porous structure. This removal process may include, for example, submerging and/or flushing the porous structure in water and/or a liquid solution to dissolve and disperse the filler material from the porous structure. The removal process may include other removal steps or techniques such as suction, vacuum cycle nucleation, sonication, vibration, physical extraction by cutting or drilling, etc., and any combination thereof to remove the filler material from the porous structure. Some of these additional removal techniques may be used on their own and/or in combination with water and/or liquid solutions as described above. In this manner, the filler material is temporarily added to the porous structure to plug the porous opening and to provide structure support that prevents deformation of the porous structure during a high-heat, high pressure injection molding process.

Body 102, 202 may be, but is not limited to being, made of any polymer such as PEEK, carbon fiber reinforced PEEK, PAEK, metals, ceramics, combinations of the foregoing, or other suitable materials that are biocompatible and possess sufficient strength and rigidity. The porous structure may be, but is not limited to, being made of any of titanium, titanium alloys, stainless steel, cobalt chrome alloys, tantalum and niobium or other suitable materials including PEEK, PAEK, or ceramics.

It is to be understood that the disclosure set forth herein includes any possible combinations of the particular features set forth above, whether specifically disclosed herein or not. For example, where a particular feature is disclosed in the context of a particular aspect, embodiment, arrangement, or configuration, that feature can also be used to the extent possible, in combination with and/or in the context of other particular aspects, embodiments, arrangements, and configurations of the technology, and in the technology in general.

Furthermore, although the technology here has been described with reference to particular features and figures, it is to be understood that these features are merely illustrative of the principles and applications of the present technology. It is therefore to be understood that numerous modifications, including changes in the sizes of the various features described herein, may be made to the illustrative arrangement and that other arrangements may be devised without departing from the spirit and scope of the present technology. In this regard, the present technology encompasses numerous additional features in addition to those specific features set forth in the claims below. Moreover, the foregoing disclosure should be taken by way of illustration rather than by way of limitation as the present technology is defined by the claims set forth below.

## Claims

1. A method of manufacturing an implant, comprising:
additively manufacturing the implant from a first material, the implant defining at least one pore;
inserting a filler material into the at least one pore after the implant has been additively manufactured;
overmolding the implant with a second material after the filler material has been inserted into the at least one pore; and
dissolving the filler material from the at least one pore prior to implanting the implant.

2. The method of claim 1, wherein the dissolving step includes submerging the implant into water.

3. The method of claim 1 or claim 2, further including a step of removing any second material covering the filler material after the dissolving step.

4. The method of any one of claims 1 to 3, wherein the inserting step includes packing the filler material into the at least one pore.

5. The method of any one of claims 1 to 4, wherein the inserting step includes filling the at least one pore completely with the filler material.

6. The method of any one of claims 1 to 5, wherein the filler material includes a soluble salt or the first material is a biocompatible metal.

7. The method of any one of claims 1 to 6, wherein the filler material is a ceramic material.

8. The method of claim 7, wherein the ceramic material includes calcium sulfate and/or magnesium sulfate.

9. The method of any one of claims 1 to 8, wherein the second material is poly-ether ether ketone (PEEK).

10. The method of any one of claims 1 to 9, wherein the overmolding step uses an injection molding technique to apply the second material.

11. The method of any one of claims 1 to 10, wherein the second material is overmolded on one side of a porous structure of the implant.

12. The method of claim 12, wherein the porous structure is a bone ingrowth surface especially adapted to facilitate regrowth of bone tissue.

13. The method of any one of claims 1 to 12, wherein the dissolving step includes submerging the implant in a liquid solution.

14. The method of any one of claims 1 to 13, wherein the filler material is adapted to withstand temperatures greater than 350° C and dissolvable in a liquid.

15. The method of any one of claims 1 to 14, wherein the overmolding step includes injecting the second material at a pressure ranging between 300 bar to 2,000 bar.
